(19) Europäisches Patentamt
European Patent Office
Office européen des brevets

(11) Veröffentlichungsnummer: **0 033 435**
**A2**

# (12) EUROPÄISCHE PATENTANMELDUNG

(21) Anmeldenummer: **81100018.1**

(22) Anmeldetag: **05.01.81**

(51) Int. Cl.³: **C 10 M 3/10**
C 07 C 13/50

(30) Priorität: **16.01.80 DE 3001363**

(43) Veröffentlichungstag der Anmeldung:
**12.08.81 Patentblatt 81/32**

(84) Benannte Vertragsstaaten:
**BE DE FR GB IT NL SE**

(71) Anmelder: **BASF Aktiengesellschaft**
**Carl-Bosch-Strasse 38**
**D-6700 Ludwigshafen(DE)**

(72) Erfinder: **Bronstert, Klaus, Dr.**
**Gartenstrasse 26**
**D-6719 Carlsberg(DE)**

(72) Erfinder: **Nickl, Johann, Dr.**
**Paray-le-Monial-Strasse 9**
**D-6702 Bad Duerkheim(DE)**

(72) Erfinder: **Ochs, Wolfram, Dr.**
**Hans-Willmann-Strasse 6**
**D-6706 Wachenheim(DE)**

(72) Erfinder: **Starke, Klaus**
**Halbergstrasse 5**
**D-6719 Weisenheim(DE)**

(54) **Schmierölgemische die als wesentlichen Bestandteil in Form eines Grundöls Cyclohexane und/oder Dekaline enthalten und Verfahren zu ihrer Herstellung.**

(57) Schmierölgemische mit verbesserter Oxydationsbeständigkeit die als wesentlichen Bestandteil in Form eines Grundöls Cyclohexane und/oder Dekaline enthalten oder daraus bestehen, die überwiegend durch ein bis drei Alkylreste gemäß den Formeln

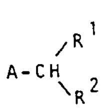 , 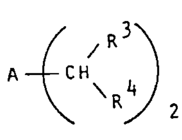 oder 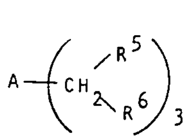

substituiert sind, in der $R^1$ bis $R^6$ Alkylreste mit 1 bis 20 Kohlenstoffatomen bedeuten, die zu mehr als 90% unverzweigt sind.

EP 0 033 435 A2

# Schmierölgemische die als wesentlichen Bestandteil in Form eines Grundöls Cyclohexane und/oder Dekaline enthalten und Verfahren zu ihrer Herstellung

Die Erfindung betrifft Schmierölgemische, die als wesentlichen Bestandteil Mono-, Di- oder Trialkylcyclohexane bzw. -dekaline enthalten.

Neben den konventionellen Motorschmierölen auf der Basis von raffinierten Mineralölen werden vermehrt solche auf der Basis von synthetischen Verbindungen hergestellt und eingesetzt.

Als eine Gruppe derartiger synthetischer Verbindungen sind Alkylbenzole bekannt und in ihren Eigenschaften beschrieben worden (z.B. I.A.C. KRULISCH, H.V. LOWTHER und B.J. MILLER, Soc. of Automotive Engineers, Fuels and Lubricants Meeting, Tulsa/USA 1977 (Nr. 770634).

Alkylbenzole als Motorschmieröle weisen u.a. die unbefriedigenden Eigenschaften auf, daß sie im Verbrennungsmotor zur thermischen und oxidativen Zersetzung unter Rußabscheidung und Bildung von Ablagerungen neigen.

Die Aufgabe der Erfindung bestand deshalb darin, Grundöle für Schmierölformulierungen zu entwickeln, die diese Nachteile nicht aufweisen.

Es wurde nun gefunden, daß diese Aufgabe gelöst wurde mit Schmierölgemischen, die als wesentlichen Bestandteil in Form eines Grundöls Cyclohexane und/oder Dekaline enthalten oder daraus bestehen, die gegebenenfalls eine oder zwei Methylgruppen tragen und wobei die Cyclohexane bzw. Dekaline (A) überwiegend durch Alkylreste gemäß den Formeln

Hp/BL

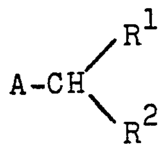 , 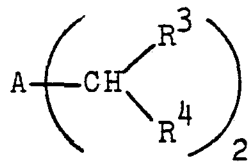 oder 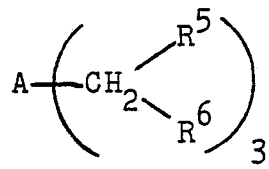

- bei mehreren Resten an verschiedenen C-Atomen des Cyclohexan- bzw. Decalinrings - substituiert sind, in der $R^1$ und $R^2$ Alkylreste mit 1 bis 20, vorzugsweise 1 bis 16 Kohlenstoffatomen bedeuten, die zu mehr als 90 % unverzweigt sind und, die Summe der Kohlenstoffatome von $R^1$ und $R^2$ 15 bis 21, vorzugsweise 16 bis 20 beträgt,

$R^3$ und $R^4$ Alkylreste mit 1 bis 18 Kohlenstoffatomen bedeuten, die zu mehr als 90 % unverzweigt sind, die Summe der Kohlenstoffatome von $R^3$ und $R^4$ 9 bis 19, vorzugsweise 12 bis 16 beträgt und wobei im Gemisch der Unterschied der Kettenlänge $R^3$-CH-$R^4$ im Durchschnitt nicht mehr als 3 Kohlenstoffatome beträgt und $R^5$ und $R^6$ Alkylreste mit 1 bis 20 Kohlenstoffatomen bedeuten, die zu mehr als 90 % unverzweigt sind, die Summe der Kohlenstoffatome von $R^5$ und $R^6$ 11 bis 21, vorzugsweise 13 bis 17 beträgt und wobei im Gemisch der Unterschied der Kettenlänge $R^5$-CH-$R^6$ im Durchschnitt nicht mehr als 3 Kohlenstoffatome beträgt.

Derartige Polyalkylcycloalkane bzw. Polyalkyldecaline zeigen teilweise verbesserte Pour Points und in allen Fällen verbesserte oxidative und thermische Beständigkeit sowie geringere Verdampfungsverluste bei guten Viskositätsindices und sonstigen für die Verwendung als Schmiermittel in Verbrennungsmotoren wichtigen Eigenschaften. Von diesen Alkylcycloalkanen sind die im wesentlichen mono- und disubstituierten Verbindungen mit 2 $-CH\langle^{R^3}_{R^4}$-Resten bevorzugt. Von den Alkyldekalinen sind die monosubstituierten Verbindungen bevorzugt.

Die Alkylcyclohexane bzw. Dekaline können hergestellt werden durch Hydrierung von Alkylbenzolen bzw. Alkyltetralinen oder Alkylnaphthalinen. Geeignete Verbindungen entstehen z.B. durch Umsetzung von einheitlichen oder statistisch chlorierten Monochlor-n-Paraffinen oder einfach ungesättigten n-Olefinen mit statistischer Verteilung der Doppelbindung mit einer C-Zahl zwischen 10 und 22, mit Alkylbenzolen bzw. Alkyltetralinen oder Naphthalinen, bevorzugt jedoch Benzol, in Gegenwart eines Friedel-Crafts-Katalysators nach bekannten Methoden. Im Intersse optimaler Eigenschaften der Alkylcyclohexane ist es dabei wichtig, daß für die Umsetzung möglichst reine Ausgangsverbindungen verwendet werden, insbesondere, daß die n-Chlorparaffine bzw. n-Olefine weniger als 5 % difunktionelle Anteile enthalten und zumindest 90 % linearen Aufbau besitzen. Ferner sollte die C-Zahl der verwendeten Chlorparaffine bzw. Olefine eine enge Verteilung aufweisen und insbesondere nicht mehr als ± 1 von der C-Zahl der Hauptfraktion abweichen, d.h. die Zahl der Kohlenstoffatome sollte sich nicht um mehr als 3 unterscheiden. Besonders geeignet als aromatische Kupplungskomponente ist Benzol. Es ergibt in den entsprechenden alkylierten Cyclohexanen im Vergleich zu den homologen mono- oder di-methylierten Benzolen die besten Eigenschaften. Trotzdem kann es in einem oder anderen Fall sinnvoll sein, als Kupplungskomponente auch Alkylbenzole, z.B. Toluol oder Xylole und für mit einer langkettigen Alkylgruppe monosubstituierte Verbindungen Naphthalin, das methylsubstituiert sein kann, zu verwenden.

Die Umsetzung wird mit der 1/2- bis 3-fach molaren Menge der Monochlorparafine unter Wahl der entsprechenden Kettenlängen vorgenommen, um zu den oben spezifizierten Produkten zu gelangen.

Dabei können auch jegliche Molverhältnisse gewählt werden, die zwischen 1/2 und 3 Mol der Monochlorparafine liegen.

Bevorzugt ist jedoch eine solche Wahl der Molverhältnisse, daß überwiegend eine Mono- oder Dialkylierung eintritt. Im Fall der Dekalinderivate ist die Monoalkylierung bevorzugt.

Für die Hydrierung der Alkylaromaten werden an sich bekannte Verfahren angewandt. Man kann mit oder ohne Lösungsmittel arbeiten. Gute Lösungsmittel sind z.B. Hexan, Heptan, Cyclohexan und andere Aliphaten bzw. Cycloaliphaten, sowie Ester oder Alkohole. Als Katalysatoren kommt bevorzugt Raney Nickel in Betracht. In diesem Falle erreicht man z.B. bei 200°C, 200 bis 300 bar und innerhalb von 5 bis 8 Stunden eine vollständige Hydrierung. Allgemein wurde beobachtet, daß die Hydrierungszeiten und der notwendige Druck mit der C-Zahl der n-Alkylgruppen der substituierten Benzole zunehmen. Als Katalysator ist gleichermaßen ein Niederschlag von 5 % Rhodium auf Aluminiumoxid geeignet, mit dem schon bei 80°C und 50 bis 180 atü in 5 bis 10 Stunden vollständige Hydrierung erreicht werden kann. Die hier angeführten Möglichkeiten sollen nur Beispiele für mögliche Ausführungsformen der Hydrierung sein. Es sind zahlreiche weitere Alternativen denkbar. Im einzelnen wird hier auf folgende Literaturstellen verwiesen: R.L. Augustine (1965, London und New York) Catalytic Hydrogenation, Seite 71; P.N. Rylander (1967, New York und London) Catalytic Hydrogenation over Pt-Metals, Seite 309; und F. Zymalkowski (1965) Katalytische Hydrierung, Seite 178.

Eine ausführliche Darstellung der katalytischen Hydrierung von Aromaten findet sich bei C. Ellis, Hydrogenation of Organic Substances, 3rd edition, D. van Nostrand Co.,

New York 1930; H. Adkins, Reactions of Hydrogen with Organic Compounds over Chromium Oxide and Nickel Catalysts, University of Wisconsin Press, Madison, Wisconsin 1937; V. Ipatieff, Catalytic Reaction at High Pressures and Temperatures, Macmillan Company, New York 1936; S. Berkman, J.C. Morell and G. Egloff, Catalysis, Reinhold Publishing Corporation, New York 1940; sowie bei K.N. Campbell and B.K. Campbell, in: Chem. Reviews 31, 156-167 (1942); und E.B. Maxted, in: G.M. Schwab, Handbuch der Katalyse VII, 1. Hälfte, 685-708, Springer-Verlag, Wien (1943).

Durch geeignete Wahl der Molverhältnisse sowie der Kohlenstoff-Zahl der linearen Monochlorparaffine bzw. Olefine bei der Alkylierung der Benzole bzw. Naphthaline lassen sich innerhalb gewisser Grenzen die Eigenschaften der Alkylcyclohexane bzw. Dekaline variieren. Verwendet man für die Dialkylierung z.B. $C_{10}$-n-Alken bzw. $C_{10}$-n-Chlorparaffin, so erhält man vergleichsweise niedrige absolute Viskosität und einen sehr guten Pour Point, jedoch unter Inkaufnahme eines mäßigen Viskositätsindexes und erhöhter Flüchtigkeit. Geht man an die obere Grenze des Bereiches der C-Zahlen der n-Alkene bzw. Chloralkane von 20, so erhält man gute Viskositätsindices sowie geringe Flüchtigkeiten bei Dialkylcyclohexanen, jedoch liegen die Pour Points und die absoluten Viskositäten höher. Man wird also je nach den gewünschten Eigenschaften der fertigen Schmierölformulierungen Grundöle entsprechend der vorliegenden Erfindung auswählen, deren Eigenschaften durch geeignete Kupplungskomponenten festgelegt werden. Die generellen Zusammenhänge sind aus den Beispielen 1-7 zu entnehmen.

Reine Dialkylcyclohexane (Beispiele 8-12) zeigen vergleichsweise zu den in den Beispielen 1-7 beschriebenen Alkylcyclohexangemischen niedrigere Viskositäten und Pour

Points sowie höhere Flammpunkte, wobei die Viskositäts-Indices in vergleichbarer Höhe liegen. Die Herstellung bestimmter Mischungen aus Alkylcyclohexanen verschiedener Substitutionsgrade, insbesondere aus Di- und Trialkylcyclohexanen stellt eine weitere Möglichkeit dar, die Eigenschaften von Schmierölen den Anforderungen anzupassen.

Die hier beschriebenen synthetischen Öle sind aufgrund ihrer guten rheologischen und schmiertechnischen Eigenschaften geeignet als Basis-Öle für voll- oder teilsynthetische Ölformulierungen für Verbrennungsmotoren. Derartige Formulierungen können Alkylcyclohexane der beschriebenen Art (neben üblichen Additiven) bis zu ca. 95 % oder in Abmischung mit Estern oder raffinierten Mineralölen enthalten. Als Additive werden je nach Beanspruchung bekannte und bewährte Stoffe, z.B. Oxidationsinhibitoren, Antikorrosionsmittel, Dispersants, Detergents oder Viskositätsindex-Verbesserer zugesetzt. (Eine Übersicht der Additiv-Klassen für Schmieröle und ihrer Funktion gibt der Report Nr. 125, "Synthetic Lubricants" des Stanford Research Institut vom Mai 1979.)

## Beispiel 1

Aus einem Monochlorundecangemisch mit statistischer Verteilung der Chloratome, das mindestens zu 99 % aus Monoverbindungen besteht, und Benzol wird nach Friedel Crafts mit $AlCl_3$ ein Gemisch von Alkylbenzolen hergestellt, das nach Abdestillieren niedriger siedender Bestandteile im Rotationsverdampfer bei $140^{\circ}C$/0,05 mbar besteht aus ca.: 7 % Mono-, 56 % Di-, 31 % Tri- und 6 % Tetraundecylbenzol; die Anteile werden bestimmt aus der Molekulargewichtsverteilung (GPC). An diesem Öl werden die in Tabelle 1 angegebenen Werte gemessen.

120 g dieses so gewonnenen Di-undecyl-benzolgemischs und 5 g Rhodium-Hydrierungs-Katalysator, der 5 % Rhodium auf $Al_2O_3$ enthält, werden in einem 150 ml-Hydrierautoklav bei 25°C mit 100 bar Wasserstoff beschickt. Nach dem Aufheizen des Autoklaveninhaltes auf 80°C wird der $H_2$-Druck auf 180 bar eingestellt. Nach 5 Stunden Rühren unter diesen Bedingungen erhält man ein farbloses klares Öl, in dem IR-spektroskopisch keine aromatischen und ungesättigten Verbindungen mehr nachzuweisen sind. Das hydrierte Produkt hat die in Tabelle 1 aufgeführten Eigenschaften.

Tabelle 1

| Bezeichnung der Verbindung | Di-undecyl-cyclohexan | Di-undecyl-benzol |
|---|---|---|
| Länge der Alkylketten | $C_{11}$ | $C_{11}$ |
| $V_{40°C}$ (cST) | 34,88 | 20,24 |
| $V_{100°C}$ (cST) | 5,67 | 4,09 |
| VI (Dean + Davies) ISO 2909 | 101 | 106 |
| Pour Point (°C) DIN 51 597 | - 55 | - 57 |
| Flammpunkt (°C) DIN 51 597 | 224 | 214 |

Beispiel 2

Aus einem Monochlor-tridecangemisch mit statistischer Verteilung der Chloratome, das zu 99 % aus Monochlorverbindungen besteht, und Benzol wird nach Friedel Crafts mit $AlCl_3$ ein Gemisch von Alkylbenzolen hergestellt, das nach Abdestillieren niedriger siedender Bestandteile im Rotationsverdampfer bei 150°C/0,05 mbar besteht aus ca.: 7 % Mono-, 53 % Di-, 33 % Tri- und 6 % Tetratridecylbenzol; die Anteile werden bestimmt aus der GPC-Molekulargewichtsverteilung. An diesem Öl werden die in Tabelle 2 angegebenen Werte gemessen.

Das Produkt und 8,9 g Raney-Nickel werden in einem 150 ml-Hydrierautoklaven bei 25°C mit 100 bar Wasserstoff beschickt. Nach dem Aufheizen auf 180°C wird der $H_2$-Druck auf 180 bar eingestellt. Nach 6 Stunden Rühren unter diesen Bedingungen wird ein farbloses, klares Öl erhalten, in dem IR-spektroskopisch keine aromatischen und ungesättigten Verbindungen mehr nachzuweisen sind. Das hydrierte Produkt hat die in Tabelle 2 aufgeführten Eigenschaften.

Tabelle 2

| Bezeichnung der Verbindung | Di-tridecyl-cyclohexan | Di-tridecyl-benzol |
|---|---|---|
| Länge der Alkylketten | $C_{13}$ | $C_{13}$ |
| $V_{40^\circ C}$ (cSt) | 56,42 | 33,47 |
| $V_{100^\circ C}$ (cSt) | 8,0 | 5,93 |
| VI (Dean + Davies) ISO 2909 | 110 | 122 |
| Pour Point (°C) DIN 51 597 | - 50 | - 18 |
| Flammpunkt (°C) DIN 51 597 | 218 | 218 |
| Oxidative und thermische Beständigkeit (Wolf-Teststreifen prüfung; Vornorm DIN 51 392; Noten 0 - 10) | 6,0 | 4,0 |
| Verdampfungsverlust (%; Wolf-Teststreifenprüfung, Vornorm DIN 51 392) | 40 | 41 |

Beispiel 3

Aus einem Monochlor-tetradecangemisch, das mindestens zu 99 % aus Monochlorverbindungen besteht, und Benzol wird nach Friedel Crafts mit $AlCl_3$ ein Gemisch von Alkylben-

zolen hergestellt, das nach Abdestillieren niedriger siedender Bestandteile im Rotationsverdampfer bei 160°C/0,05 mbar besteht aus ca.: 10 % Mono-, 56 % Di-, 27 % Tri- und 4 % Tetra-(tetradecylbenzol); die Anteile werden bestimmt aus der GPC-Molekulargewichtsverteilung. An diesem Öl wurden die in Tabelle 3 angegebenen Werte gemessen.

120 g dieses so gewonnenen Di-tetradecyl-benzolgemischs wird mit Rhodium-Katalysator unter den in Beispiel 1 beschriebenen Bedingungen hydriert, bis IR-spektroskopisch keine aromatischen und ungesättigten Verbindungen mehr nachzuweisen sind. Das hydrierte Produkt hat die in Tabelle 3 aufgeführten Eigenschaften.

Tabelle 3

| Bezeichnung der Verbindung | Di-tetradecyl-cyclohexan | Di-tetradecyl-benzol |
|---|---|---|
| Länge der Alkylketten | $C_{14}$ | $C_{14}$ |
| $V_{40^{\circ}C}$ (cSt) | 58,7 | 31,88 |
| $V_{100^{\circ}C}$ (cSt) | 8,49 | 5,85 |
| VI (Dean + Davies) ISO 2909 | 117 | 128 |
| Pour Point (°C) DIN 51 597 | - 45 | - 15 |
| Flammpunkt (°C) DIN 51 597 | 230 | 228 |
| Oxidative und thermische Beständigkeit (Wolf-Teststreifenprüfung; Vornorm DIN 51 392; Noten 0-10) | 6,5 | 4,0 |
| Verdampfungsverlust (%; Wolf-Teststreifenprüfung; Vornorm DIN 51 392) | 33 | 37 |

Beispiel 4

Aus einem Monochlor-pentadecangemisch, das mindestens zu 99 % aus Monochlorverbindungen besteht, und Benzol werden nach Friedel Crafts mit $AlCl_3$ ein Gemisch von Alkylbenzolen hergestellt, das nach Abdestillieren niedriger siedender Bestandteile im Rotationsverdampfer bei 160°C/0,05 mbar besteht aus ca.: 10 % Mono-, 65 % Di-, 21 % Tri- und 3 % Tetra-pentadecylbenzol; die Anteile werden bestimmt aus der GPC-Molekulargewichtsverteilung. An diesem Öl werden die in Tabelle 4 angegebenen Werte gemessen.

120 g dieses so gewonnenen Di-pentadecylbenzolgemischs wird mit Rhodium-Katalysator unter den in Beispiel 1 beschriebenen Bedingungen hydriert bis IR-spektroskopisch keine aromatischen und ungesättigten Verbindungen mehr nachzuweisen sind. Das hydrierte Produkt hat die in Tabelle 4 aufgeführten Eigenschaften.

Tabelle 4

| Bezeichnung der Verbindung | Di-pentadecyl-cyclohexan | Di-pentadecyl-benzol |
|---|---|---|
| Länge der Alkylketten | $C_{15}$ | $C_{15}$ |
| $V_{40^{o}C}$ (cSt) | 48,82 | 36,62 |
| $V_{100^{o}C}$ (cSt) | 7,56 | 6,57 |
| VI (Dean + Davies) ISO 2909 | 120 | 135 |
| Pour Point (°C) DIN 51 597 | - 39 | - 10 |
| Flammpunkt (°C) DIN 51 597 | 244 | 236 |
| Oxidative und thermische Beständigkeit (Wolf-Teststreifenprüfung; Vornorm DIN 51 392; Noten 0 - 10) | 7 | 4,5 |
| Verdampfungsverlust (%; Wolf-Teststreifenprüfung; Vornorm DIN 51 392) | 31 | 39 |

Beispiel 5

Aus einem Monochlor-hexadecangemisch, das mindestens zu 99 % aus den Monochlorverbindungen bestand, und Benzol wird nach Friedel Crafts mit $AlCl_3$ ein Gemisch von Alkylbenzolen hergestellt, das nach Abdestillieren niedriger siedender Anteile im Rotationsverdampfer bei 180°C/ 0,05 mbar besteht aus ca.: 5 % Mono-, 60 % Di-, 33 % Tri- und 2 % Tetra-hexadecylbenzol; die Anteile werden bestimmt aus der GPC-Molekulargewichtsverteilung. An diesem Öl werden die in Tabelle 5 angegebenen Werte gemessen.

75 g dieses so gewonnenen Di-hexadecylbenzolgemischs, 30 g n-Heptan und 6 g Rhodium-Katalysator mit 5 % Rhodium auf $Al_2O_3$ werden unter den in Beispiel 1 beschriebenen Bedingungen hydriert, bis IR-spektroskopisch keine aromatischen und ungesättigten Verbindungen mehr nachzuweisen waren. Das hydrierte Produkt hat die in Tabelle 5 aufgeführten Eigenschaften.

Tabelle 5

| Bezeichnung der Verbindung | Di-hexadecyl-cyclohexan | Di-hexadecyl-benzol |
|---|---|---|
| Länge der Alkylketten | $C_{16}$ | $C_{16}$ |
| $V_{40^\circ C}$ (cSt) | 83,53 | 46,1 |
| $V_{100^\circ C}$ (cSt) | 11,24 | 7,73 |
| VI (Dean + Davies) ISO 2909 | 123 | 136 |
| Pour Point (°C) DIN 51 597 | - 27 | ± 0 |
| Flammpunkt (°C) DIN 51 597 | 229 | 234 |
| Oxidative und thermische Beständigkeit (Wolf-Teststreifenprüfung; Vornorm DIN 51 392; Noten 0 - 10) | 7,5 | 4,5 |
| Verdampfungsverlust (%; Wolf-Teststreifenprüfung; Vornorm DIN 51 392) | 25 | 34 |

Beispiel 6

Aus einem Monochlor-octadecan-Gemisch, das zu 99 % aus den Monochlorverbindungen besteht, und Benzol wird nach Friedel Crafts mit $AlCl_3$ ein Gemisch von Alkylbenzolen

hergestellt, das nach Abdestillieren niedriger siedender Anteile im Rotationsverdampfer bei 200°C/0,05 mbar besteht aus ca.:13 % Mono-, 74 % Di-, 12 % Tri- und 1 % Tetra-octadecylbenzol; die Anteile werden bestimmt aus der GPC-Molekulargewichtsverteilung. An diesem Öl wurden die in Tabelle 6 angegebenen Werte gemessen.

80 g dieses so gewonnenen Öls, 40 g n-Heptan und 6 g Rhodium-Katalysator mit 5 % Rhodium auf $Al_2O_3$ werden in einem Hydrierautoklaven bei 25°C mit 100 bar Wasserstoff beschickt. Nach dem Aufheizen des Autoklaveninhalts auf 120°C wird der $H_2$-Druck auf 250 bar eingestellt. Nach 10 Stunden Rühren unter diesen Bedingungen erhält man ein farbloses, klares Öl, in dem IR-spektroskopisch keine aromatischen und ungesättigten Verbindungen mehr nachzuweisen sind. Das hydrierte Produkt hat die in Tabelle 6 aufgeführten Eigenschaften.

Tabelle 6

| Verbindung<br>Länge der Alkylketten | Di-octadecyl-cyclohexan<br>$C_{18}$ | Di-octadecyl-benzol<br>$C_{18}$ |
|---|---|---|
| $V_{40°C}$ (cSt) | 72,74 | 46,96 |
| $V_{100°C}$ (cSt) | 10,33 | 8,05 |
| VI (Dean + Davies) ISO 2909 | 127 | 145 |
| Pour Point (°C) DIN 51 597 | - 5 | - 6 |
| Flammpunkt (°C) DIN 51 597 | 234 | 216 |
| Oxidative und thermische Beständigkeit (Wolf-Teststreifen prüfung; Vornorm DIN 51 392; Noten 0-10) | 8,0 | 6,5 |
| Verdampfungsverlust (%; Wolf-Teststreifenprüfung, Vornorm DIN 51 392) | 25 | - 31 |

Beispiel 7

Aus einem Monochlor-pentadecangemisch, das mindestens zu 99 % aus den Monochlorverbindungen besteht, und Toluol wird nach Friedel Grafts mit $AlCl_3$ ein Gemisch von Alkylbenzolen hergestellt, das nach Abdestillieren niedriger siedender Anteile im Rotationsverdampfer bei

160°C/0,05 mbar besteht aus ca.:10 % Mono-, 85 % Di- und 5 % Tri-pentadecyltoluol; die Anteile werden bestimmt aus der GPC-Molekulargewichtsverteilung. An diesem Öl werden die in Tabelle 7 angegebenen Werte gemessen.

120 g dieses so gewonnenen Di-pentadecyltoluols werden in einem 150-ml-Hydrierautoklav mit 6 g Rhodium-Hydrierkatalysator (5 % Rhodium auf $Al_2O_3$) bei 25°C mit 100 bar Wasserstoff beschickt. Nach dem Aufheizen auf 90°C wird der $H_2$-Druck auf 180 bar eingestellt. Nach 6,5 h Rühren unter diesen Bedingungen wird ein farbloses klares Öl erhalten, in dem IR-spektroskopisch keine aromatischen und ungesättigten Verbindungen mehr nachzuweisen sind. Das hydrierte Produkt hat die in Tabelle 7 aufgeführten Eigenschaften.

Tabelle 7

| Bezeichnung der Verbindung | Di-pentadecyl-methyl-cyclohexan | Di-pentadecyl-toluol |
|---|---|---|
| Länge der Alkylketten | $C_{15}$ | $C_{15}$ |
| $V_{40^{o}C}$ (cSt) | 57,15 | 35,28 |
| $V_{100^{o}C}$ (cSt) | 7,82 | 5,97 |
| VI (Dean + Davies) ISO 2909 | 100 | 114 |
| Pour Point (°C) DIN 51 597 | - 38 | - 30 |
| Flammpunkt (°C) DIN 51 597 | 220 | 225 |
| Oxidative und thermische Beständigkeit (Wolf-Teststreifenprüfung; Vornorm DIN 51 392; Noten 0 - 10) | 6,0 | 4,0 |
| Verdampfungsverlust [x] (%; Wolf-Teststreifen-Prüfung; Vornorm DIN 51 392) | 34 | 38 |

[x] Beschreibung der "Wolf-Teststreifen-Prüfung" Vornorm DIN 51 392

Das zu prüfende Öl wird in einer Umlaufapparatur, in der es in Luftatmosphäre über einen Stahlblechstreifen fließt, 6 Stunden bei 250°C gehalten. Nach Abkühlung des Öls auf Raumtemperatur wird dieser Vorgang unter den gleichen Bedingungen wiederholt.

Die Beurteilung erfolgt visuell anhand der auf dem Blechstreifen abgelagerten Bestandteile durch Noten von 0 - 10, wobei die Note 10 die beste Note darstellt.

Die Prüfung erlaubt eine Beurteilung der oxidativen und thermischen Beständigkeit von Motorölen.

Gleichzeitig wird in dieser Prüfung eine Beurteilung des Verdampfungsverlustes (in Gew.%) vorgenommen.

Beispiel 8 - 12

Gemäß den Beispielen 2 - 6 werden jeweils Gemische von Alkylbenzolen hergestellt, die überwiegend die entsprechenden Dialkylbenzole enthalten. Daraus wurden durch fraktionierte Destillation die einzelnen Dialkylbenzolfraktionen, nämlich Ditridecyl-, Ditetradecyl-, Dipentadecyl-, Dihexadecyl- und Dioctadecylbenzol, in einer Reinheit von 97 99 % isoliert (Bestimmung der Zusammensetzung durch die GPC-Molekulargewichtsverteilung). Durch anschließende Hydrierung werden daraus reine Dialkylcyclohexane gewonnen, deren Eigenschaften in den Tabellen 8 - 12 wiedergegeben sind.

Tabelle 8

| Bezeichnung der Verbindungen | Di-tridecyl-cyclohexan | Di-tridecyl-benzol |
|---|---|---|
| Länge der Alkylketten | $C_{13}$ | $C_{13}$ |
| $V_{40^\circ C}$ (cSt) | 42,33 | 24,8 |
| $V_{100^\circ C}$ (cSt) | 6,46 | 4,77 |
| VI (Dean + Davies) ISO 2909 | -10 | 116 |
| Pour Point (°C) DIN 51 597 | -51 | -48 |
| Flammpunkt (°C) DIN 51 597 | 220 | 211 |
| Verdampfungsverlust (Gew.-%) Noack-Test DIN 51 581 | 8 | 10 |
| Oxidative und thermische Beständigkeit (Wolf-Teststreifenprüfung; Vornorm DIN 51 392; Noten 0-10) | 6,5 | 4,5 |
| Verdampfungsverlust (%; Wolf-Teststreifen-prüfung, Vornorm DIN 51 392) | 33 | 36 |

Tabelle 9

| Bezeichnung der Verbindungen | Di-tetradecyl-cyclohexan | Di-tetradecyl-benzol |
|---|---|---|
| Länge der Alkylketten | $C_{14}$ | $C_{14}$ |
| $V_{40^oC}$ (cSt) | 44,72 | 27,52 |
| $V_{100^oC}$ (cSt) | 6,89 | 5,19 |
| VI (Dean + Davies) ISO 2909 | 115 | 120 |
| Pour Point (°C) DIN 51 597 | -47 | -45 |
| Flammpunkt (°C) DIN 51 597 | 235 | 220 |
| Verdampfungsverlust (Gew.-%) Noack-Test DIN 51 581 | 5 | 8 |
| Oxidative und thermische Beständigkeit (Wolf-Teststreifen prüfung; Vornorm DIN 51 392; Noten 0-10) | 6,5 | 4,0 |
| Verdampfungsverlust (%; Wolf-Teststreifen-prüfung, Vornorm DIN 51 392) | 28 | 33 |

Tabelle 10

| Bezeichnung der Verbindungen Länge der Alkylketten | Di-pentadecyl-cyclohexan $C_{15}$ | Di-pentadecyl-benzol $C_{15}$ |
|---|---|---|
| $V_{40^\circ C}$ (cSt) | 51,7 | 34,57 |
| $V_{100^\circ C}$ (cSt) | 7,72 | 6,08 |
| VI (Dean + Davies) ISO 2909 | 118,0 | 123 |
| Pour Point (°C) DIN 51 597 | -45 | -30 |
| Flammpunkt (°C) DIN 51 597 | 242 | 233 |
| Verdampfungsverlust (Gew.-%) Noack-Test DIN 51 581 | 3 | 6 |
| Oxidative und thermische Beständigkeit (Wolf-Teststreifenprüfung; Vornorm DIN 51 392; Noten 0-10) | 7,0 | 5,0 |
| Verdampfungsverlust (%; Wolf-Teststreifenprüfung, Vornorm DIN 51 392) | 23 | 30 |

Tabelle 11

| Bezeichnung der Verbindungen<br>Länge der Alkylketten | Di-hexadecyl-cyclohexan<br>$C_{16}$ | Di-hexadecyl-benzol<br>$C_{16}$ |
|---|---|---|
| $V_{40^\circ C}$ (cSt) | 58,93 | 37,03 |
| $V_{100^\circ C}$ (cSt) | 8,62 | 6,66 |
| VI (Dean + Davies)<br>ISO 2909 | 125 | 137 |
| Pour Point (°C)<br>DIN 51 597 | -39 | -18 |
| Flammpunkt (°C)<br>DIN 51 597 | 253 | 244 |
| Verdampfungsverlust (Gew.-%)<br>Noack-Test DIN 51 581 | 2 | 5 |
| Oxidative und thermische Beständigkeit (Wolf-Teststreifenprüfung; Vornorm DIN 51 392; Noten 0-10) | 7,0 | 5,5 |
| Verdampfungsverlust (%; Wolf-Teststreifenprüfung, Vornorm DIN 51 392) | 17 | 25 |

Tabelle 12

| Bezeichnung der Verbindungen | Di-octadecyl-cyclohexan | Di-octadecyl-benzol |
|---|---|---|
| Länge der Alkylketten | $C_{18}$ | $C_{18}$ |
| $V_{40^\circ C}$ (cSt) | 68,75 | 49,46 |
| $V_{100^\circ C}$ (cSt) | 9,97 | 8,25 |
| VI (Dean + Davies) ISO 2909 | 129 | 141 |
| Pour Point (°C) DIN 51 597 | -15 | -3 |
| Flammpunkt (°C) DIN 51 597 | 274 | 260 |
| Verdampfungsverlust (Gew.-%) Noack-Test DIN 51 581 | 1 | 3 |
| Oxidative und thermische Beständigkeit (Wolf-Teststreifen prüfung; Vornorm DIN 51 392; Noten 0-10) | 8,5 | 6,5 |
| Verdampfungsverlust (%; Wolf-Teststreifen-prüfung, Vornorm DIN 51 392) | 8 | 16 |

Beispiel 13

Aus einem Monochlortetradecangemisch mit statistischer Verteilung der Chloratome, das mindestens zu 99 % aus

Monoverbindungen besteht, und Benzol wird nach Friedel-Crafts mit $AlCl_3$ ein Gemisch von überwiegend Trialkylbenzolen hergestellt, woraus durch Fraktionierung in einer Vakuumkolonne die Trialkylbenzol-Fraktion isoliert wurde, die zu 99 % aus Tri-tetradecyl-benzol bestand. Die Anteile werden bestimmt aus der Molekulargewichtsverteilung (GPC). An diesem Öl wurde für die oxidative und thermische Beständigkeit ein Benotungswert von 5,5 gefunden (Wolf-Teststreifenprüfung; Vornorm DIN 51 392; Noten 0 bis 10).

120 g dieses so gewonnenen Tri-tetradecyl-benzols und 5 g Rhodium-Hydrierungs-Katalysator, der 5 % Rhodium auf $Al_2O_3$ enthält, werden in einem 150 ml-Hydrierautoklav bei 25°C mit 100 bar Wasserstoff beschickt. Nach dem Aufheizen des Autoklaveninhaltes auf 180°C wird der $H_2$-Druck auf 200 bar eingestellt. Nach 5 Stunden Rühren unter diesen Bedingungen erhält man ein farbloses klares Öl, in dem IR-spektroskopisch keine aromatischen und ungesättigten Verbindungen mehr nachzuweisen sind. Das hydrierte Produkt hat die in Tabelle 13 aufgeführten Eigenschaften.

Tabelle 13

| Bezeichnung der Verbindung | Tri-tetradecyl-cyclohexan |
|---|---|
| Länge der Alkylkette | $C_{14}$ |
| $V_{40°C}$ (cSt) | 116,1 |
| $V_{100°C}$ (cSt) | 13,52 |
| VI (Dean + Davies) ISO 2909 | 108 |
| Pour Point (°C) DIN 51 597 | -47 |
| Flammpunkt (°C) DIN 51 597 | 285 |
| Oxidative und thermische Beständigkeit (Wolf-Teststreifenprüfung; Vornorm DIN 51 392; Noten 0 - 10) | 7,5 |
| Verdampfungsverlust (Gew.-%) Noack-Test DIN 51 581 | 3,5 |

Beispiel 14

Aus einem Monochlor-hexadecangemisch mit statistischer Verteilung der Chloratome, das zu 99 % aus Monochlorverbindungen besteht, und Benzol wird nach Friedel-Crafts mit $AlCl_3$ ein Gemisch von überwiegend Trialkylbenzolen herge-

stellt, woraus die Trialkylbenzol-Fraktion durch Fraktionierung in einer Vakuum-Kolonne isoliert wird; diese besteht zu 99 % aus Tri-hexadecyl-benzol. Die Anteile werden bestimmt aus der GPC-Molekulargewichtsverteilung.

An diesem Öl wird für die oxidative und thermische Beständigkeit ein Benotungswert von 6,0 gefunden (Wolf-Teststreifenprüfung; Vornorm DIN 51 392; Noten 0 bis 10).

Das Produkt und 8,9 g Raney-Nickel werden in einem 150 ml-Hydrierautoklaven bei 25°C mit 100 bar Wasserstoff beschickt. Nach dem Aufheizen auf 180°C wird der $H_2$-Druck auf 280 bar eingestellt. Nach 6 Stunden Rühren unter diesen Bedingungen wird ein farbloses, klares Öl erhalten, in dem IR-spektroskopisch keine aromatischen und ungesättigten Verbindungen mehr nachzuweisen sind. Das hydrierte Produkt hat die in Tabelle 14 aufgeführten Eigenschaften.

Tabelle 14

| Bezeichnung der Verbindung | Tri-hexadecyl-cyclohexan |
|---|---|
| Länge der Alkylkette | $C_{16}$ |
| $V_{40°C}$ (cSt) | 138,5 |
| $V_{100°C}$ (cSt) | 16,4 |
| VI (Dean + Davies) ISO 2909 | 126 |
| Pour Point (°C) DIN 51 597 | -45 |
| Flammpunkt (°C) DIN 51 597 | 310 |
| Oxidative und thermische Beständigkeit (Wolf-Teststreifenprüfung; Vornorm DIN 51 392; Noten 0 10)$^{x}$ | 7,5 |
| Verdampfungsverlust (Gew.-%) Noack-Test DIN 51 581 | 2,1 |

Beispiel 15

Aus einem Monochlor-eicosan-Gemisch, das mindestens zu 99 % aus Monochlorverbindungen besteht, und Benzol wird nach Friedel-Crafts mit $AlCl_3$ ein Gemisch von überwiegend Tri-alkylbenzolen hergestellt, woraus die Trialkylbenzol--Fraktion durch Fraktionierung in einer Vakuum-Kolonne isoliert wird; diese besteht zu 97 % aus Tri-eicosylbenzol. Die Anteile werden bestimmt aus der GPC-Molekulargewichtsverteilung. An diesem Öl wird für die oxidative und thermische Beständigkeit ein Benotungswert von 6,0 gefunden (Wolf-Teststreifenprüfung; Vornorm DIN 51 392; Noten 0 bis 10)$^{x}$.

Tabelle 15

| Bezeichnung der Verbindung<br>Länge der Alkylkette | Tri-eicosyl-cyclohexan<br>$C_{20}$ |
|---|---|
| $V_{40^{\circ}C}$ (cSt) | 158,2 |
| $V_{100^{\circ}C}$ (cSt) | 19,7 |
| VI (Dean + Davies)<br>ISO 2909 | 148 |
| Pour Point (°C)<br>DIN 51 597 | -30 |
| Flammpunkt (°C)<br>DIN 51 597 | 370 |
| Oxidative und thermische Beständigkeit (Wolf-Teststreifenprüfung; Vornorm DIN 51 392; Noten 0 bis 10)$^{x}$ | 8,5 |
| Verdampfungsverlust (Gew.-%)<br>Noack-Test<br>DIN 51 581 | 1,3 |

Beispiel 16

Aus einem Monochloroctadecan-Gemisch mit statistischer Verteilung der Chloratome, das mindestens zu 99 % aus Monoverbindungen besteht, und Benzol wird nach Friedel--Crafts mit $AlCl_3$ ein Alkylbenzol hergestellt, das nach Fraktionierung in einer Vakuumkolonne zu 98 % aus Monooctadecylbenzol besteht; der Anteil wird bestimmt aus der Molekulargewichtsverteilung (GPC). An diesem Öl wurden die in Tabelle 16 angegebenen Werte gemessen.

120 g dieses so gewonnenen Mono-Octadecylbenzols und 5 g Rhodium-Hydrierungs-Katalysator, der 5 % Rhodium auf $Al_2O_3$ enthält, werden in einem 150 ml-Hydrierautoklav bei 25°C mit 100 bar Wasserstoff beschickt. Nach dem Aufheizen des Autoklaveninhaltes auf 120°C wird der $H_2$-Druck auf 180 bar eingestellt. Nach 5 Stunden Rühren unter diesen Bedingungen erhält man ein farbloses klares Öl, in dem IR-spektroskopisch keine aromatischen und ungesättigten Verbindungen mehr nachzuweisen sind. Das hydrierte Produkt hat die in Tabelle 16 aufgeführten Eigenschaften.

Tabelle 16

| Bezeichnung der Verbindung | Mono-Octadecyl-cyclohexan | Mono-Octadecyl-benzol |
|---|---|---|
| Länge der Alkylkette | $C_{18}$ | $C_{18}$ |
| $V_{40°C}$ (cSt) | 21,1 | 10,53 |
| $V_{100°C}$ (cSt) | 3,07 | 2,70 |
| VI (Dean + Davies) ISO 2909 | 112 | 90 |
| Pour Point (°C) DIN 51 597 | -30 | -50 |
| Flammpunkt (°C) DIN 51 597 | 210 | 208 |
| Verdampfungsverlust (Gew.-%) Noack-Test DIN 51 581 | 30 | 35 |
| Oxidative und thermische Beständigkeit (Wolf-Teststreifenprüfung; Vornorm DIN 51 392; Noten 0 bis 10)$^x$ | 6,5 | 4,5 |

Beispiel 17

Aus einem Monochloreicosan-Gemisch mit statistischer Verteilung der Chloratome, das zu 99 % aus Monochlorverbindungen besteht, und Benzol wird nach Friedel-Crafts mit $AlCl_3$ ein Alkylbenzol hergestellt, das nach Fraktionierung

in einer Vakuumkolonne zu 99 % aus Mono-eicosylbenzol besteht, die Anteile werden bestimmt aus der GPC-Molekulargewichtsverteilung. An diesem Öl werden die in Tabelle 17 angegebenen Werte gemessen.

Das Produkt und 9 g Raney-Nickel werden in einem 150 ml-Hydrierautoklaven bei 25°C mit 100 bar Wasserstoff beschickt. Nach dem Aufheizen auf 180°C wird der $H_2$-Druck auf 200 bar eingestellt. Nach 6 Stunden Rühren unter diesen Bedingungen wird ein farbloses, klares Öl erhalten, in dem IR-spektroskopisch keine aromatischen und ungesättigten Verbindungen mehr nachzuweisen sind. Das hydrierte Produkt hat die in Tabelle 17 aufgeführten Eigenschaften.

Tabelle 17

| Bezeichnung der Verbindung | Mono-eicosyl-cyclohexan | Mono-eicosyl-benzol |
|---|---|---|
| Länge der Alkylkette | $C_{20}$ | $C_{20}$ |
| $V_{40^oC}$ (cSt) | 15,57 | 13,44 |
| $V_{100^oC}$ (cSt) | 3,65 | 3,27 |
| VI (Dean + Davies) ISO 2909 | 128 | 112 |
| Pour Point ($^oC$) DIN 51 597 | -15 | -15 |
| Flammpunkt ($^oC$) DIN 51 597 | 232 | 223 |
| Oxidative und thermische Beständigkeit (Wolf-Teststreifenprüfung; Vornorm DIN 51 392; Noten 0 bis 10)$^x$ | 6,5 | 4,0 |
| Verdampfungsverlust Noack-Test (Gew.-%) DIN 51 581 | 17 | 23 |

Beispiel 18

Aus einem Monochlor-octadecan-Gemisch, das mindestens zu 99 % aus Monochlorverbindungen besteht, und Tetralin wird nach Friedel-Crafts mit $AlCl_3$ ein Alkyltetralin hergestellt, das nach Fraktionierung in einer Vakuumkolonne zu

98 % aus Mono-octadecyltetralin besteht; die Anteile werden bestimmt aus der GPC-Molgewichtsverteilung. An diesem Öl wurden die in Tabelle 18 angegebenen Werte gemessen.

120 g dieses so gewonnenen Mono-octadecyltetralins wird mit Rhodium-Katalysator unter den in Beispiel 1 beschriebenen Bedingungen hydriert, bis IR-spektroskopisch keine aromatischen und ungesättigten Verbindungen mehr nachzuweisen sind. Das hydrierte Produkt hat die in Tabelle 18 aufgeführten Eigenschaften.

Tabelle 18

| Bezeichnung der Verbindung | Mono-octadecyl-dekalin | Mono-octadecyl-tetralin |
|---|---|---|
| Länge der Alkylkette | $C_{18}$ | $C_{18}$ |
| $V_{40^\circ C}$ (cSt) | 36,59 | 38,55 |
| $V_{100^\circ C}$ (cSt) | 6,05 | 5,86 |
| VI (Dean + Davies) ISO 2909 | 110 | 91 |
| Pour Point (°C) DIN 51 597 | -27 | -25 |
| Flammpunkt (°C) DIN 51 597 | 235 | 228 |
| Oxidative und thermische Beständigkeit (Wolf-Teststreifenprüfung; Vornorm DIN 51 392; Noten 0 bis 10)x | 6,5 | 4,0 |
| Verdampfungsverlust (Gew.-%) Noack-Test DIN 51 581 | 7,3 | 8,8 |

## Patentansprüche

1. Schmierölgemische, die als wesentlichen Bestandteil in Form eines Grundöls Cyclohexane und/oder Dekaline enthalten oder daraus bestehen, die gegebenenfalls eine oder zwei Methylgruppen tragen, dadurch gekennzeichnet, daß die Cyclohexane bzw. Dekaline (A) überwiegend durch ein bis drei Alkylreste gemäß den Formeln

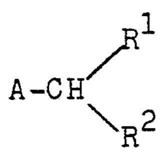 , 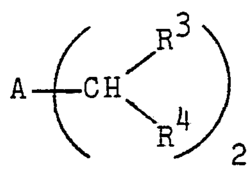 oder 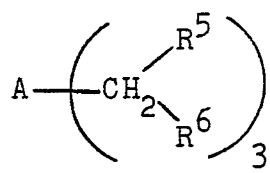

- bei mehreren Resten an verschiedenen C-Atomen des Cyclohexan- bzw. Dekalinrings - substituiert sind, in der $R^1$ und $R^2$ Alkylreste mit 1 bis 20 Kohlenstoffatomen bedeuten, die zu mehr als 90 % unverzweigt sind und die Summe der Kohlenstoffatome von $R^1$ und $R^2$ 15 bis 21 beträgt, $R^3$ und $R^4$ Alkylreste mit 1 bis 18 Kohlenstoffatomen bedeuten, die zu mehr als 90 % unverzweigt sind, die Summe der Kohlenstoffatome von $R^3$ und $R^4$ 9 bis 19 beträgt und wobei im Gemisch der Unterschied der Kettenlängen $R^3$-CH-$R^4$ im Durchschnitt nicht mehr als 3 Kohlenstoffatome beträgt und $R^5$ und $R^6$ Alkylreste mit 1 bis 20 Kohlenstoffatome bedeuten, die zu mehr als 90 % unverzweigt sind, die Summe der Kohlenstoffatome von $R^5$ und $R^6$ 11 bis 21 beträgt und wobei im Gemisch der Unterschied der Kettenlängen $R^5$-CH-$R^6$ im Durchschnitt nicht mehr als 3 Kohlenstoffatome beträgt.

2. Schmierölgemische gemäß Anspruch 1, dadurch gekennzeichnet, daß die Summe der Kohlenstoffatome von $R^1$ und $R^2$ 16 bis 20 beträgt.

3. Schmierölgemische gemäß Anspruch 1, dadurch gekennzeichnet, daß die Summe der Kohlenstoffatome von $R^3$ und $R^4$ 12 bis 16 beträgt.

4. Schmierölgemische gemäß Anspruch 1, dadurch gekennzeichnet, daß die Summe der Kohlenstoffatome von $R^5$ und $R^6$ 13 bis 17 beträgt.

5. Verfahren zur Herstellung von Grundölen für Schmierölgemische gemäß Anspruch 1, dadurch gekennzeichnet, daß man Benzol, Toluol, Xylole, Tetraline oder Naphthaline mit der ungefähr 1/2 bis 3-molaren Menge an im Durchschnitt monochlorierten Paraffinen oder Monololefinen mit 10 bis 22 Kohlenstoffatomen, wobei jeweils die Kettenlänge des einzusetzenden Chlorparaffin- oder Olefingemischs sich nicht um mehr als 3 Kohlenstoffatome unterscheidet und die Chloratome bzw. Doppelbindungen im Molekül der Chlorparaffine bzw. Olefine statistisch verteilt sind, nach Friedel Crafts mono- bis trialkyliert und die erhaltenen Alkylierungsprodukte zu den entsprechenden Cyclohexan- bzw. Dekalinderivaten hydriert.